# EUROPEAN PATENT SPECIFICATION

(11) **EP 2 651 391 B1**
(45) Date of publication and mention of the grant of the patent: **23.01.2019**
(21) Application number: 11799420.2
(22) Date of filing: 15.12.2011
(51) Int. Cl.: A61K 9/00, A61K 9/20, A61K 31/437, A61K 33/10, A61P 25/20, A61K 9/46, A61K 47/02, A61K 9/50, A61K 31/4985

(54) **PHARMACEUTICAL COMPOSITION FOR ORAL ADMINISTRATION INTENDED TO PREVENT MISUSE**
PHARMAZEUTISCHE ZUSAMMENSETZUNG ZUR ORALEN VERABREICHUNG BESTIMMT UM MISSBRAUCH ZU VERHINDERN
COMPOSITION PHARMACEUTIQUE DESTINEE A EVITER L'ABUS PAR DES TIERS

(30) Priority: 16.12.2010 FR 1060655
(43) Date of publication of application: 23.10.2013
(73) Proprietor: SANOFI, 75008 Paris (FR)
(72) Inventor: MARTINEZ, Annick, F-75013 Paris (FR); ROUGEOT, Olivier, F-75013 Paris (FR); YAGUCHI, Yoshikatsu, F-75013 Paris (FR)
(74) Representative: Taravella, Brigitte
(86) International application number: PCT/EP2011/072933
(87) International publication number: WO 2012/080408

(56) References cited:
- WO-A1-00/38649
- WO-A2-2009/071219
- WO-A2-2009/071219
- FR-A1- 2 787 715
- US-A1- 2007 196 476
- US-A1- 2007 196 476
- US-A1- 2010 204 259
- US-A1- 2010 204 259

## Description

### Field of the invention

The field of the present invention is that of oral pharmaceutical forms, the composition of which makes it possible to prevent possible misuse of the active principle present therein. The present invention thus relates to a pharmaceutical composition for oral administration intended to prevent abuse of use at the expense of a third party.

### Prior Art and Technical Problem

The active principles which may be included in the pharmaceutical compositions according to the present invention are compounds which might possibly form the object of an abuse of use. Mention may be made, as such, of the following categories of active principles: analgesics, anxiolytics or hypnotics.

Mention may thus be made, among analgesics, of methadone, among weak opiate antalgesics, of codeine and its derivatives, dextropropoxyphen or tramadol, or, among mixed opioid antalgesics, of morphine agonists/antagonists, such as buprenorphine or pentazocine, morphine and morphinomimetics, such as pethidine, dextromoramide, oxycodone, fentanyl and tamgesic.

Mention may be made, among anxiolytic compounds, of diazepam, medazepam, oxazepam, lorazepam, benzodiazepines, meprobamate, hydroxyzine or buspirone.

Mention may also be made of the antihistaminic phenergan, digitalis-like compounds, such as digoxin and digitalin, and the antivitamin K coumarin.

The hypnotic compounds can belong to any therapeutic category, whether their duration of action is short or long, by way of example:
- compounds of the category of the benzodiazepines recognized for their hypnotic activity, such as triazolam, loprazolam, nitrazepam, lormetazepam, temazepam, estazolam, flunitrazepam, brotizolam, cinolazepam, haloxazolam or doxefazepam, and their pharmaceutically acceptable salts, for example loprazolam mesylate,
- zopiclone and particularly (R)-zopiclone of the cyclopyrrolone therapeutic category,
- zaleplon of the pyrazolopyrimidine therapeutic category,
- zolpidem and its pharmaceutically acceptable salts of the imidazopyridine therapeutic category. One of the particularly preferred zolpidem salts is zolpidem hemitartrate (or also known as zolpidem tartrate).

The object of the present invention is to provide a pharmaceutical composition intended for oral administration, comprising an active principle exposed to the risk of abuse of use or one of its pharmaceutically acceptable salts, capable of preventing misuse, while guaranteeing, for the patient, a quality of treatment in accordance with his or her requirements.

The composition according to the invention makes it possible both to produce release of the active principle after oral administration in accordance with expectations and, if it is introduced into an optionally alcoholic drink, to generate visual means on contact with the latter. These visual means then make it possible to prevent the said drink from being administered to a person without his or her knowledge, the person being rapidly alerted visually by the presence of a foreign body (namely the pharmaceutical composition) in his or her drink.

Provision has already been made, in Patent Application WO 00/38649, for a pharmaceutical composition for oral administration intended to prevent the abuse of use of a pharmaceutical active principle at the expense of a third party. The visual means generated by the composition described correspond either to the floatation of the tablet, or to the formation of insoluble particles, optionally in combination with an effervescence, or to a combination of these visual means. However, the visual means consisting of the opacification of the drink as described below is neither described nor suggested. This visual means makes possible easy and rapid detection of the pharmaceutical composition in the drink, without causing trouble to or bringing about an unpleasant feeling for the patient to whom the composition according to the invention is orally administered.

### Summary of the invention

More specifically, a subject-matter of the present invention is a pharmaceutical composition for oral administration, characterized in that it comprises an active principle which might be the object of an abuse of use or of misuse, preferably a hypnotic, analgesic or anxiolytic compound or one of its pharmaceutically acceptable salts, and comprises components which, if it is introduced into an aqueous drink optionally comprising alcohol, generate visual means corresponding to an opacification of the drink and to at least one other visual means chosen from effervescence, the formation of insoluble particles, the flotation of the composition and a combination of these visual means, the components generating the said opacification of the drink comprising titanium dioxide particles.

According to a preferred embodiment of the invention, the components generating the said opacification of the drink consist of titanium dioxide particles.

Preferably, the pharmaceutical composition comprises at least 15 mg, preferably at least 20 mg, of titanium dioxide particles. Advantageously, the pharmaceutical composition comprises 15 mg of titanium dioxide particles.

According to one embodiment, the composition comprises at least one effervescence generator.

According to another specific embodiment, the effervescence generator is composed solely of a carbon dioxide generator, preferably chosen from a carbonate or a bicarbonate of an alkali metal, of an alkaline earth metal or of an amino acid, such as calcium carbonate, sodium bicarbonate, potassium bicarbonate, potassium carbonate, L-lysine carbonate, arginine carbonate, sodium sesquicarbonate and their mixture; advantageously, the carbon dioxide generator is calcium carbonate.

According to another specific embodiment, the pharmaceutical composition additionally comprises a lipophilic excipient chosen from glyceryl stearates, palmitate/stearates and behenates; hydrogenated vegetable oils and their derivatives; vegetable and animal waxes and their derivatives; hydrogenated castor oils and their derivatives; and cetyl esters and alcohols; preferably, the lipophilic excipient is glyceryl behenate.

According to another alternative form, the pharmaceutical composition additionally comprises at least one disintegrating agent preferably chosen from sodium carboxymethylstarch, crosslinked sodium carboxymethylcellulose and crosslinked polyvinylpyrrolidone, and more advantageously crosslinked polyvinylpyrrolidone.

The disintegrating agent preferably varies from 2 to 15% (inclusive) by weight, more preferably from 4 to 12% (inclusive), by weight, with respect to the total weight of the composition.

According to another alternative form, the pharmaceutical composition additionally comprises at least one soluble agent of polyol type preferably chosen from mannitol, xylitol, sorbitol, maltitol and their mixture.

Preferably, the amount of soluble agent of polyol type is between 20 and 50% by weight and more preferably between 30 and 45% by weight, with respect to the total weight of the composition.

According to a preferred embodiment, the pharmaceutical composition is for rapid disintegration in the mouth.

According to another preferred embodiment, the active principle is coated with a coating agent based on a polymer which is soluble at pH 5 or less and which is permeable at a pH above 5.

According to an alternative form, the pharmaceutical composition additionally comprises at least one hydrophilic excipient preferably chosen from cellulose, hydroxyethylcellulose, hydroxypropylcellulose, hydroxypropylmethylcellulose, carboxymethylcellulose or sodium carboxymethylcellulose derivatives; vegetable gums and their derivatives; alginic acid derivatives; polyethylene glycols and their derivatives; starches and their derivatives; silicas and their derivatives; polymethacrylates; and copolymers of acrylic and methacrylic acids.

Preferably, the pharmaceutical composition is provided in the form of a tablet.

According to a preferred alternative form, the active principle is in the form of granules, more preferably with a mean particle diameter of between 100 and 300 µm, advantageously between 100 and 250 µm.

The active principle which might form the object of an abuse of use is preferably a hypnotic compound; the hypnotic compound preferably belongs to the category of the benzodiazepines, cyclopyrrolones, pyrazolopyrimidines or imidazopyridines.
in the specific case where the active principle is a hypnotic compound, it is preferably chosen from triazolam, loprazolam, nitrazepam, lormetazepam, temazepam, estazolam, flunitrazepam, brotizolam, cinolazepam, haloxazolam, doxefazepam, zopiclone, (R)-zopiclone, zaleplon or zolpidem and their pharmaceutically acceptable salts. More preferably, the hypnotic compound is zolpidem or one of its pharmaceutically acceptable salts. More preferably still, the zolpidem salt is zolpidem hemitartrate.

The invention also relates to a pharmaceutical tablet comprising **(1)** an internal phase comprising coated granules based on an active principle which might form the object of an abuse of use or one of its pharmaceutically acceptable salts, in particular a hypnotic compound, and a coating agent based on a polymer which is soluble at pH 5 or less and which is permeable at a pH above 5, **(2)** an external phase comprising components which, if the tablet is introduced into an aqueous drink which optionally comprises alcohol, generate visual means corresponding to an opacification of the drink and to at least one other visual means chosen from effervescence of the tablet, the formation of insoluble particles, the flotation of the tablet and a combination of these visual means, the components generating the said opacification of the drink comprising titanium dioxide particles, preferably consisting of titanium dioxide particles, and the said external phase additionally comprising at least one disintegrating agent and optionally at least one soluble agent of polyol type.

The composition according to the invention exhibits the advantage of having a release and an action of the active principle which are not detrimentally affected by the presence of components intended to be revealed during a possible misuse.

In the context of the present invention, the pharmaceutical compositions are prolonged release, controlled release or, preferably immediate release pharmaceutical compositions.

### Brief Description of the Figures

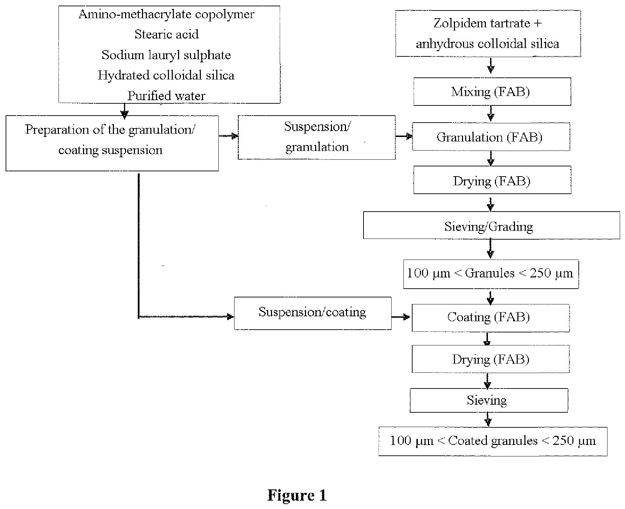

The main stages of the preparation of coated granules according to specific form of the invention are presented in Figure 1 (FAB means Fluidized Air Bed).

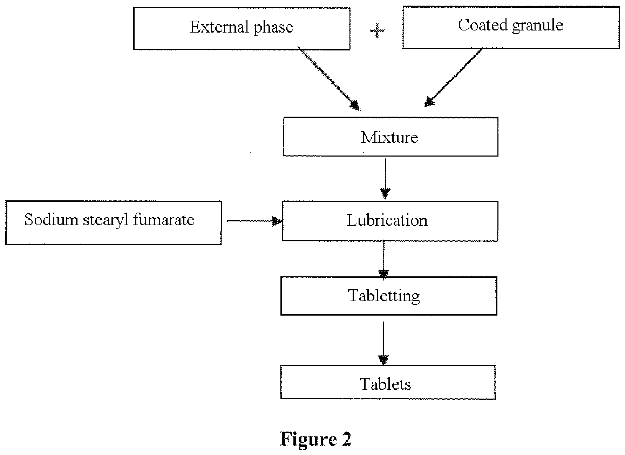

The main final stages of the preparation of tablets according to a specific form of the invention are presented in Figure 2.

### Detailed Description of the Invention

According to the specific form of the invention, the composition is for rapid disintegration in the mouth (or also known as orodispersible composition). In this context and advantageously, the composition according to the invention reveals its antimisuse properties when it is introduced into a drink but does not reveal them in the mouth.

In the context of the present invention, the term "visual means" means any element which indicates the presence of the composition according to the invention in an aqueous drink optionally comprising alcohol and which can take the forms, for example, of an opacity, of an effervescence, of a dye, of flotation of the composition at the surface of the drink or of formation of insoluble particles at the surface of the drink, on the edges of the receptacle, in the drink and/or on the bottom of the receptacle.

The aqueous drink can be transparent, translucent or opaque, can be coloured or colourless and can optionally comprise alcohol. It can in particular correspond to coffee, tea, wine, spirits, champagne, sake, any fizzy drink, such as a fizzy drink of cola type, optionally with the addition of alcohol, any other carbonated beverage, with or without alcohol, or any cocktail or liquid mixture based on fruit juice, on alcohols, and the like. The composition according to the invention is particularly suitable for transparent or translucent drinks.

The composition intended for oral administration according to the invention is provided in the form of a tablet or any other solid formulation form. The composition according to the present invention can be provided in the form of a conventional (single-layer) tablet or a multilayer tablet, in particular of two or three layers. When the pharmaceutical composition is for controlled release or prolonged release, the composition can be a multilayer tablet, it being possible for one layer to exhibit a specific disintegrating time different from that of another layer. The composition can also be in the form of conventional hard galatin capsules (comprising powder or comprising microgranules) or of sachets comprising powder or granules. Preferably, the composition according to the invention is a tablet.

The pharmaceutical compositions according to the present invention have a size acceptable for conventional oral administration. Thus, preference is given to the compositions with a weight of less than 800 mg, preferably of less than 500 mg and more particularly of less than 400 mg (such as, for example, compositions having a weight of 100 mg, 150 mg, 200 mg, 250 mg or 400 mg).

At least one of the components of the composition according to the invention generates an opacification of the drink when the composition is introduced into an aqueous drink optionally comprising alcohol. This opacification of the drink is in particular advantageously obtained by the presence of titanium dioxide (TiO₂) in the composition.

The TiO₂ is more specifically white in colour. It is provided in the form of particles. These particles are generally insoluble in water and organic solvents. When the composition, preferably the tablet, breaks up in a drink, the TiO₂ particles present in the composition then form a suspension in the drink which is particularly visible by the consumer of the drink.

The TiO₂ particles are in the form of an odourless and tasteless white powder. The particles measure approximately 0.3 µm and generally at most 1 µm and often form aggregates of approximately 100 µm. The titanium dioxide is sold in particular by Brenntag under the name Titanium Dioxide 3328 ®. The TiO₂ particles preferably exhibit a specific surface of greater than 10 m²/g and advantageously of less than 200 m²/g. More specifically, the specific surface of the TiO₂ particles is greater than 10 and less than 15 m²/g.

More particularly, the composition according to the invention, in particular in the tablet form, comprises at least 15 mg or at least 20 mg, of TiO₂ particles (such as, for example, 15, 30 or 60 mg per tablet). This minimal amount makes possible detection in a receptacle,such as a glass or a cup, containing a drink (the volume of the drink generally being at most 250 ml, preferably 200 or 75 ml, and even 4 cl). In the composition, the amount of titanium dioxide particles is preferably between 8 and 30% by weight, with respect to the total weight of the composition. Advantageously, the amount of titanium dioxide particles is preferably between 10 and 25% by weight, with respect to the total weight of the composition (such as, for example, 12, 15 or 20%).

According to a specific form of the invention, the visual means correspond at least to opacification and to effervescence. According to this form, the composition according to the invention preferably comprises an active principle which might form the object of an abuse of use, preferably a hypnotic, analgesic or anxiolytic compound or one of its pharmaceutically acceptable salts, titanium dioxide particles and at least one effervescence generator, preferably an effervescence generator composed solely of an agent which generates carbon dioxide.

One of the other visual means is thus effervescence (when the composition is introduced into an aqueous drink optionally comprising alcohol). Effervescence is generally obtained by means of an effervescence generator, such as described below. The flotation of the tablet (or of its disintegrated particles) can optionally be provided by the effervescence.

The effervescence generator can be provided either in the form of an effervescent pair, composed of an agent which generates carbon dioxide and a pharmaceutically acceptable acid compound, or composed solely of an agent which generates carbon dioxide, the acidity of the drink making possible effervescence.

The agent which generates carbon dioxide is usually a carbonate or a bicarbonate of an alkali metal, of an alkaline earth metal or of an amino acid. Mention may be made, for example, as the agent which generates carbon dioxide, of calcium carbonate, sodium bicarbonate, potassium bicarbonate, potassium carbonate, L-lysine carbonate, arginine carbonate, sodium sesquicarbonate or their mixture. Preference is given, as the agent which generates carbon dioxide, to calcium carbonate.

The pharmaceutically acceptable acid compound is an acid anhydride, a monocarboxylic acid, a polycarboxylic acid or a polycarboxylic acid partial salt. The pharmaceutically acceptable acid compound can more particularly be chosen from citric acid, tartaric acid, ascorbic acid, fumaric acid, nicotinic acid, acetylsalicylic acid, malic acid, adipic acid, succinic acid, glutaric anhydride, citric anhydride, maleic acid, malonic acid, monosodium citrate and succinic anhydride.

Preference is given, as effervescence-generating pair, to the effervescent pair composed of calcium carbonate and anhydrous citric acid.

The agent which generates carbon dioxide can be composed of a mixture of several abovementioned agents which generate carbon dioxide.

In the effervescence generator, the content of acid compound is generally chosen so that the ratio of the number of moles of acid compound to the number of moles of carbon dioxide generator is between 1 and 2.

According to a preferred form, the effervescence generator is composed solely of an agent which generates carbon dioxide. According to this form, the composition according to the invention is devoid of pharmaceutically acceptable acid compound. According to this form, the agent which generates carbon dioxide can be one of those mentioned above and is preferably calcium carbonate.

This is because it has been found that the majority of the drinks used in the context of misuse are acidic (pH of less than 7) and that they thus make it possible for effervescence to take place solely in the presence of agent which generates carbon dioxide. In addition, this form exhibits the advantage of not having the phenomenon of the effervescence which happens in the mouth during the oral administration of the composition, the mouth generally being of a non-acid pH.

In this context, the amount of effervescence-generating agent is preferably between 5 and 25% by weight, advantageously between 10 and 20% (inclusive) by weight, more specifically 15% by weight, with respect to the total weight of the composition.

The composition can release visible insoluble particles even if the tablet does not float or does not immediately float.

According to a specific form, in order to provide for the formation of insoluble particles, other than titanium dioxide, visible at the surface of the drink and/or on the walls of the receptacle, the composition or in particular the tablet also comprises one or more lipophilic excipients.

Mention may be made, among the lipophilic excipients, of glyceryl stearates, palmitate/stearates and behenates; hydrogenated vegetable oils and their derivatives; vegetable and animal waxes and their derivatives; hydrogenated castor oils and their derivatives; and cetyl esters and alcohols. Preference is given, among lipophilic excipients, to glyceryl behenate.

The amount of lipophilic excipients in the composition can vary within a wide range, in particular as a function of the active principle, of the other components present in the composition and of their respective amounts. To give an order of magnitude and advantageously, the amount of lipophilic excipients can vary from 0.05 to 15% (inclusive) by weight, preferably from 0.1 to 6% by weight, advantageously from 0.2 to 5% (inclusive) (for example: 0.25, 3 or 4%) by weight, with respect to the total weight of the composition.

The composition, in particular the tablet, in the liquid or at its surface, can break up, in particular under the action of effervescence. As break up occurs, the formation of agglomerates is observed, which agglomerates float and possibly become stuck to the walls of the receptacle and/or become suspended in the liquid.

According to a specific form of the invention, the visual means correspond at least to opacification, effervescence and to the formation of insoluble particles. According to this form, the composition according to the invention comprises an active principle which might form the object of an abuse of use, preferably a hypnotic, analgesic or anxiolytic compound or one of its pharmaceutically acceptable salts, titanium dioxide particles, at least one effervescence generator, preferably an effervescence generator composed solely of an agent which generates carbon dioxide, and at least one lipophilic excipient.

The insoluble particles can thus be obtained by the presence of a lipophilic excipient in the composition according to the invention.

The composition, in particular the tablet, can additionally exhibit viscosity properties which appear on contact with any drink. This characteristic makes possible trapping of the gas possibly produced by the effervescence which is also reflected by a swelling of the composition and in particular of the tablet. The fall in density generated then makes it possible to provide for the maintenance of the tablet at the surface of the drink and thus makes possible flotation of the composition or of its disintegrated particles. Such a viscosity can be obtained by virtue of one or more gelifiable compounds.

The gelifiable compound can comprise one or more hydrophilic excipients which bring about the swelling of the composition (and in particular of the tablet) and optionally the trapping of the gas formed by the effervescence generator. The gelifiable compound can correspond to a mixture of lipophilic and hydrophilic excipients.

The composition according to the invention in addition optionally comprises at least one hydrophilic excipient. Mention may be made, among hydrophilic excipients, of derivatives of cellulose, hydroxyethylcellulose, hydroxypropylcellulose (molecular weight of from 50 to 1250 kDa), hydropropylmethylcellulose (molecular weight of from 10 to 1500 kDa), carboxymethylcellulose and sodium carboxymethylcellulose; vegetable gums and their derivatives; alginic acid derivatives; polyethylene glycols and their derivatives; starches and their derivatives; silicas and their derivatives; polymethacrylates; and copolymers of acrylic and methacrylic acids.

At least one of the constituents of the gelifiable compound, either the hydrophilic excipient or the lipophilic excipient, can be chosen so as to have little solubility in alcohol.

Thus, the composition according to the invention, in particular the tablet, which has little solubility in an alcoholic liquid or drink with a degree of greater than 45, gives the liquid or the drink the appearance of a suspension of insoluble viscous particles.

Finally, another visual means, such as a colorant, can advantageously be added to the composition according to the invention. This is of particular use when the drink is a translucent drink or fruit juice. This colorant can thus colour the drink. The colorant is generally neither black nor white.

Mention may be made, among the colorants which can be used in the tablet according to the invention, of indigotin, cochineal red, orange yellow S, allura red AC, iron oxides, cucurmin, riboflavin, tartrazine, quinoline yellow, azorubin, amaranth, carmines, erythrosine, red 2G, patent blue V, brilliant blue FCF, chlorophylls, cupric complexes of chlorophylls, green S, caramels, brilliant black BN, vegetable charcoal, brown FK and HT, carotenes, annatto extracts, paprika extracts, lycopene, lutein, canthaxanthin, beetroot red, anthocyanins, litholrubin BK or any other colorants suitable for consumption.

Other additives can advantageously complete the composition of the tablet.

Thus, a disintegrating agent can be added, such as sodium carboxymethylstarch (molecular weight of from 500 to 1000 kDa), such as the product sold by Avebe under the trade name Primojel® or the product sold by JRS Pharma under the trade name Explotab®, crosslinked sodium carboxymethylcellulose, also denoted by the term sodium croscarmellose, or crosslinked polyvinylpyrrolidone or crospovidone, such as the product sold by BASF under the trade name Kolidon® CL. Crosslinked polyvinylpyrrolidone is very particularly preferred.

Finally, the technical preparation of the tablets can also lead to the introduction of tabletting agents:
- lubricating agents, such as magnesium stearate, stearic acid, glyceryl monostearate, polyethylene glycols having a molecular weight of from 400 to 7 000 000, hydrogenated castor oil, glyceryl behenate, mono-, bi- or trisubstituted glycerides, or sodium stearyl fumarate, sold under the name of Pruv® by JRS Pharma;
- flow agents, such as colloidal silica or any other silica, for example the product sold by Degussa under the trade name Aerosil;
- binders, such as starch, buffers, absorbents, diluents, such as lactose, and any other pharmaceutically acceptable additive.

As specified above, according to a specific form of the invention, the composition is for rapid disintegration in the mouth (or also known as orodispersible composition). In this context and advantageously, the composition according to the invention reveals its antimisuse properties when it is introduced into a drink but does not reveal them in the mouth.

The term "rapid disintegration in the mouth" is understood to mean a tablet which breaks up in less than 3 minutes, preferably in less than 30 seconds (for example in 15, 20 or 30 seconds). Breaking up can be measured according to the test described in section 2.9.1 of the European Pharmacopoeia, using a device of Sotax DT3 type (the test can be carried out with or without disc(s); preferably, discs are employed for compositions in the form of film-coated tablets, whereas the test is preferably carried out without disc for the orodispersible tablet forms).

In this context, the composition according to the invention comprises at least one disintegrating agent such as those mentioned above and in particular sodium carboxymethylstarch, crosslinked sodium carboxymethylcellulose or crosslinked polyvinylpyrrolidone, and more advantageously crosslinked polyvinylpyrrolidone.

The amount of the disintegrating agent in the composition can vary within wide limits, in particular as a function of the active principle, of the other components present in the composition and of their respective amounts. To give an order of magnitude and advantageously, the amount of the disintegrating agent can vary from 2 to 15% (inclusive) by weight, preferably from 4 to 12% (inclusive) by weight and advantageously from 7 to 10% (inclusive) (for example: 10%) by weight, with respect to the total weight of the composition.

The composition according to the invention can additionally comprise at least one soluble agent of polyol (or sugar alcohol) type. A polyol (or sugar alcohol) is a hydrogenated form of sugar in which the carbonyl group (i.e., aldehyde or ketone) has been reduced to give a primary or secondary hydroxyl group. In particular, the diluting soluble agent is chosen from a polyol of less than 13 carbon atoms, preferably of from 5 to 12 carbon atoms (inclusive), this polyol preferably being chosen from the group consisting of mannitol, xylitol, sorbitol, maltitol and their mixture. Preferably, mannitol is used. The said soluble agent is used in the form of granules, of powders or of a mixture of granules and powders. The granules generally exhibit a mean diameter of from 100 to 500 micrometres; in the form of a powder, the soluble agent exhibits a mean particle diameter generally of less than 100 micrometres, in particular between 10 and 90 micrometres.

The amount of the soluble agent in the composition can vary within wide limits, in particular as a function of the active principle, of the other components present in the composition and of their respective amounts. To give an order of magnitude and advantageously, the amount of the diluting soluble agent having binding properties can vary from 20 to 50% (inclusive) by weight, preferably from 30 to 45% (inclusive) (for example: 31, 32, 33, 34, 35, 42 and 43%) by weight, with respect to the total weight of the composition.

Thus, when the composition is for rapid disintegration in the mouth, the composition in addition advantageously comprises at least one disintegrating agent and at least one diluting agent of polyol type.

According to an alternative form of the invention, the active principle in the composition according to the invention is coated, encapsulated and/or pretreated, in order to mask the unpleasant taste of the active principle. This is because active principles, such as zolpidem and in particular zolpidem tartrate, have a bitter taste which may have to be masked. Various technologies, such as in particular granulation, coating, optionally in a fluidized air bed, and the like, can be employed in the context of the masking of the taste of active principles where the taste is unpleasant to the patient. The compounds for masking the taste which are used in the context of these technologies can be polymers: namely cellulose derivatives (HPC, HPMC, EC), methacrylic acid derivatives (Eudragit range) and polyvinyl acetate derivatives. It is also possible to envisage microencapsulation by a supercritical CO₂ phase process or by a simple or complex coacervation method. The same abovementioned masking polymers can be used, along with other excipients of chitosan, alginate or gelatin type. "Hot" (spray drying) or "cold" (spraying cooling) atomization processes can also be employed to mask the taste of an active principle. The "spray cooling" technology is described in WO2004/058137 and consists in melting a fatty matrix and in incorporating the active principle therein. The latter is either dissolved or dispersed in the fatty phase, composed of fatty acids, or esters of fatty acids, or fatty alcohols, or waxes, or mixture thereof. The molten mixture is subsequently brought, via a peristaltic pump, up to a twin-fluid nozzle where it is atomized into more or less fine droplets (depending on the parameters applied). Finally, the droplets are cooled by a stream of cold air in the atomization chamber and converted into solid granules comprising the active principle dispersed throughout the fatty matrix. It is also possible to envisage the use of a hot melt coating process. A hot melt coating process is in particular that which consists in preparing an active principle composition comprising (a) a granulated centre composed of active principle grains agglomerated in the presence of binder, preferably a hydrophilic agent chosen from hydrophilic polymers or heat-fusible agents (such as cellulose derivatives (hydroxypropylcellulose), povidone (polyvinylpyrrolidone), sucrose, gums, starches, gelatin or macrogols (polyethylene glycols)), and (b) a coating layer for the said granulated centre composed of a fatty matrix (such as stearic acid, palmitic acid or myristic acid, pure or as mixtures, and/or their corresponding fatty alcohols). This technology is described in particular in Patent Application FR1053034, filed on 21 April 2010 by the Applicant Company.

These technologies make it possible to obtain pharmaceutical compositions with correct taste masking while providing for dissolution of the active principle in an acidic medium which is not slowed down.

According to a specific embodiment of the invention, the active principle is coated with a coating agent based on a polymer which is soluble at pH 5 or less and which is permeable at a pH above 5. Mention may in particular be made, as such, of amino-methacrylate copolymers of E type sold by Evonik® under the trade mark Eudragit® E, such as Eudragit® E100 (in the form of granules) or Eudragit® EPO (in the form of powders). Preference is very particularly given to the amino-methacrylate copolymer in the form of powders (such as Eudragit® EPO).

The active principle participating in the composition according to the invention is generally provided in the form of crystals having a mean diameter of approximately 100 µm. Advantageously, in particular if it is coated, the active principle is granulated beforehand in order to preferably exhibit a mean particle diameter of between 100 and 300 µm, advantageously between 100 and 250 µm. The granulation can be carried out, for example, according to a conventional technique by mixing the different compounds, in order to obtain a homogeneous powder, wetting with an alcoholic, aqueous or aqueous/alcoholic solution and then drying, in a fluidized air bed, until a predetermined residual moisture percentage is obtained. The granules are subsequently preferably sieved, in order to recover the granules exhibiting a mean particle diameter of between 100 and 300 µm, advantageously between 100 and 250 µm. The solution advantageously comprises a binder, such as, for example, polyvinylpyrrolidone (molecular weight of from 28 to 1500 kDa), hydroxypropylmethylcellulose (or hypromellose), an amino-methacrylate copolymer of E type as described above or any other pharmaceutically acceptable binder. It is preferable to use the amino-methacrylate copolymer.

The coating of the active principle is carried out by conventional techniques, such as, for example, by mixing the granules of active principle as obtained above with a solution comprising the coating agent as defined above, followed by drying in a fluidized air bed. The solution thus comprises the coating agent, composed of at least one polymer which is soluble at pH 5 or less and which is permeable at a pH above 5; in addition it comprises at least one surface-active agent, at least one plasticizer, at least one anti-blocking agent, at least one solvent and preferably a mixture of these components. Preferably, the amount of dry matter of the solution to be deposited on the granules is between 50 and 99% by weight, advantageously from 60 to 70% (inclusive) by weight, with respect to the total weight of the coated granule. Preferably, the amount of the coating agent in the solution is between 50 and 70% by weight, more particularly between 60 and 65% by weight, with respect to the total weight of the coated granule.

A tablet according to the present invention can be obtained by any conventional tabletting technology known to a person skilled in the art by mixing powders and/or granulating using, for example, rotary presses. Thus, the components of the composition according to the invention are mixed with one another either in solution or in the form of powders and/or granules, in order subsequently to be optionally dried, then tabletted. The mixture necessary for the tabletting can be obtained by dry granulation or direct mixing of the constituents. The constituents are thus those specified above, in particular the active principle, and in particular in the form of granules, optionally coated, encapsulated and/or pretreated, as described above, titanium dioxide particles, one or more other component(s) against misuse, and optionally other constituents, such as those specified above in the text, such as, for example, a disintegrating agent, a soluble agent of polyol type, or also lubricating agents, flow agents and/or binders.

A tablet can take a cylindrical, lenticular, spheroidal, ovoidal or other shape which makes possible easy administration and easy swallowing.

In addition, a film coating can be applied to the tablet according to the invention, which film coating is thus found on the external surface of the tablet. This film coating is intended in particular for the protection of the active principle, for example with regard to light, or is also intended to hide colorants present in the internal phase of the composition. This coating can more particularly be envisaged when the composition is not orodispersible. This film can have a base composed of a polymeric material, such as ethylcellulose, hydroxypropylcellulose or hydroxypropylmethylcellulose, and of an opacifying agent, such as titanium dioxide.

This film can in addition be supplemented by a plasticizer and/or lubricant, such as a polyethylene glycol (for example of 400 type), and optionally coloured by a colorant, such as iron oxide or indigotin lake.

The active principle capable of an abuse of use is, according to a specific form of the invention, a hypnotic compound; the hypnotic compound preferably belongs to the category of the benzodiazepines, cyclopyrrolones, pyrazolopyrimidines or imidazopyridines. It is more particularly chosen from triazolam, loprazolam, nitrazepam, lormetazepam, temazepam, estazolam, flunitrazepam, brotizolam, cinolazepam, haloxazolam, doxefazepam, zopiclone, (R)-zopiclone, zaleplon or zolpidem and their pharmaceutically acceptable salts. In particular, the hypnotic agent is zolpidem or one of its salts and more specifically zolpidem hemitartrate (or also known as zolpidem tartrate). According to another form, the hypnotic agent is (R)-zopiclone.

The amounts of active principle necessary are the same as those which are usually administered.

By way of example, the amount of zolpidem hemitartrate is between 2 and 10 mg, such as, for example, 2.5, 5 and 10 mg.

A specific embodiment of the tablet according to the invention is, for example, a tablet comprising **(1)** an internal phase comprising coated granules based on an active principle which might form the object of an abuse of use or one of its salts, as described above, in particular a hypnotic compound, and a coating agent based on a polymer which is soluble at pH 5 or less and which is permeable at a pH above 5, **(2)** an external phase comprising components which, if the tablet is introduced into an aqueous drink which optionally comprises alcohol, generate visual means corresponding to an opacification of the drink and to at least one other visual means chosen from effervescence of the tablet, the flotation of the tablet, the formation of insoluble particles and the combination of these visual means, the components generating the said opacification of the drink comprising titanium dioxide particles, preferably consisting of titanium dioxide particles, and the said external phase additionally comprising at least one disintegrating agent and optionally at least one soluble agent of polyol type.

Preferably, the external phase comprises titanium dioxide particles, at least one lipophilic excipient and preferably an agent which generates carbon dioxide. Advantageously, the external phase or more generally the tablet is devoid of pharmaceutically acceptable acid compound.

According to a specific form, the tablet according to the invention comprises an external phase comprising titanium dioxide particles, calcium carbonate and glyceryl behenate which is devoid of pharmaceutically acceptable acid compound.

This specific embodiment is particularly advantageous for the reasons described above, namely the tablet according to the invention is capable, if it is introduced into a drink optionally comprising alcohol, of generating visual means on contact with the latter, it reveals its antimisuse properties when it is introduced into a drink but it does not reveal them in the mouth, and the release and the action of the active principle, when it is administered orally, are in accordance with expectations. The release and the action of the active principle are not detrimentally affected by the presence, in the tablet, of the constituents intended to be revealed during a possible misuse.

According to a specific form, the tablet according to the invention comprises from 0.1 to 40% by weight of active principle, preferably from 1 to 5% by weight of active principle when zolpidem or one of its pharmaceutically acceptable salts is involved, comprises at least 15 mg of titanium dioxide particles per tablet, comprises from 5 to 25% by weight, preferably from 10 to 20% by weight, of effervescence generator, comprises from 0.05 to 15% (inclusive), preferably from 0.1 to 6%, of lipophilic excipients, comprises from 2 to 15% (inclusive), preferably from 4 to 12% (inclusive), of disintegrating agent and comprises from 20 to 50%, preferably from 30 to 45%, of soluble agent of polyol type, the percentages being expressed with respect to the total weight of the tablet.

Another subject-matter of the present invention is a therapeutic treatment method, comprising the oral administration to a subject requiring it of at least one composition according to the invention. In the case of hypnotic active principles, such as zolpidem, the therapeutic method is intended in particular to treat insomnia, in particular occasional, transient or chronic insomnia.

Finally, the present invention relates to the composition according to the invention for use in a therapeutic treatment method, the said composition making it possible to prevent the abuse of use thereof. In the case of the hypnotic active principles, such as zolpidem, the therapeutic treatment is in particular a treatment of insomnia, in particular occasional, transient or chronic insomnia.

The subject is more specifically a mammal and very particularly a human being.

The following examples illustrate the present invention without wishing, however, to limit the scope thereof.

### Examples

### Example 1

### Orodispersible zolpidem tartrate tablets, dosage 10 mg

### Manufacturing process (see Figures 1 and 2):

Zolpidem tartrate is mixed with anhydrous colloidal silica in a fluidized bed. The mixture is subsequently granulated in a fluidized bed using a suspension of amino-methacrylate copolymer. This suspension is preprepared by dispersing amino-methacrylate copolymer, sodium lauryl sulphate, stearic acid and hydrated colloidal silica in purified water, according to the recommendations of the supplier of amino-methacrylate copolymer.

The zolpidem tartrate/anhydrous colloidal silica mixture is granulated by spraying, by top spray, the suspension based on amino-methacrylate copolymer and is then dried.

The dry granule obtained is graded and sieved, so as to select the particles having a size of between 100 µm and 250 µm.

The granules selected are subsequently coated with this same suspension of amino-methacrylate copolymer in a fluidized bed equipped with a Wurster column (bottom spray spraying process). Once the amount of suspension has been deposited, the granule is dried and then sieved, so as to retain only the particles having a size of between 100 µm and 250 µm.

**Table 1**

| **Composition** | **Unit formula (mg)** | **Percentage formula (%)** |
|---|---|---|
| Zolpidem tartrate | 10.00 | 4.00 |
| Anhydrous colloidal silica (Aerosil® 200, sold by Evonik Degussa GmbH®) | 0.20 | 0.08 |
| Amino-methacrylate copolymer (Eudragit® EPO, sold by Evonik Degussa GmbH®) | 13.90 | 5.56 |
| Sodium lauryl sulphate (sold by Cognis®) | 1.38 | 0.55 |
| Stearic acid (Stearic Acid 2236®, sold by Mallinckrodt Baker®) | 2.06 | 0.83 |
| Hydrated colloidal silica (Syloid® FP 244, sold by GRACE Davison®) | 4.82 | 1.93 |
| Mannitol (Pearlitol® 160C, sold by Roquette®, and/or Parteck M200, sold by Merck®) | 95.14 | 34.06 |
| Crosslinked polyvinylpyrrolidone (Kollidon® CL, sold by ISP®) | 25.00 | 10.00 |
| Aspartame (sold by Ajinomoto Sweeteners Europe SAS) | 3.75 | 1.50 |
| Sodium stearyl fumarate (Pruv®, sold by JRS Pharma®) | 3.75 | 1.50 |
| Titanium dioxide (Titanium Dioxide 3328®, sold by Cognis®) | 30.00 | 12.00 |
| Microcrystalline cellulose (Avicel® PH 101, sold by FMC Biopolymer®) | 25.00 | 10.00 |
| Glyceryl behenate (Compritol 888 ATO®, sold by Gattefosse®) | 7.50 | 3.00 |
| Calcium carbonate (Hubercal® 150, sold by Hubert | 37.50 | 15.00 |
| Total | 250.00 | 100.00 |

The coated granule obtained is subsequently mixed in a tumbler mixer with the external phase comprising mannitol, crosslinked polyvinylpyrrolidone, aspartame, titanium dioxide particles, glyceryl behenate and calcium carbonate. Finally, the mixture is lubricated using sodium stearyl fumarate in the tumbler mixer.

The mannitol used is either in the form of a powder (Pearlitol 160C), of granules (Parteck M200) or of a mixture of powder and granules; in particular, the ratios of powder/granules percentages by weight varying from 60/40-100/0 were tested.

The final mixture obtained is tabletted on a rotary tabletting press.

The composition is described in detail in Table 1.

Breaking up was measured according to the test described in section 2.9.1 of the European Pharmacopoeia, using a device of Sotax DT3 type. The test was carried out without the disc. The result obtained is 13 s.

The antimisuse effectiveness was evaluated by introducing a tablet into different drinks. The results obtained are presented in the following Table 2.

**Table 2**

| **Drink evaluated** | **Appearance of the drink after introduction of the tablet** | **Visual detection of the tablet** |
|---|---|---|
| Water | Cloudy liquid with white suspended particles | Yes |
| Orange juice | White particles floating at the surface of the drink | Yes |
| Fizzy drink of cola type | Significant effervescence of the fizzy drink with presence of white particles in the foam | Yes |
| Wine | Presence of floating white particles at the surface of the liquid | Yes |
| Whisky | Cloudiness of the liquid with presence of white suspended particles | Yes |

### Example 2

### Orodispersible zolpidem tartrate tablets, dosage 5 mg

The orodispersible zolpidem tartrate tablets, dosed at 5 mg, are manufactured according to the same manufacturing process as that described in Example 1.

The composition is described in detail in the following Table 3.

**Table 3**

| **Composition** | **Unit formula (mg)** | **Percentage formula (%)** |
|---|---|---|
| Zolpidem tartrate | 5.00 | 2.50 |
| Anhydrous colloidal silica (Aerosil® 200, sold by Evonik Degussa GmbH®) | 0.10 | 0.05 |
| Amino-methacrylate copolymer (Eudragit® EPO, sold by Evonik Degussa GmbH®) | 6.93 | 3.47 |
| Sodium lauryl sulphate (sold by Cognis®) | 0.68 | 0.34 |
| Stearic acid (Stearic Acid 2236®, sold by Mallinckrodt Baker®) | 1.03 | 0.51 |
| Hydrated colloidal silica (Syloid® FP 244, sold by GRACE Davison®) | 2.40 | 1.20 |
| Mannitol (Pearlitol® 160C, sold by Roquette®, and/or Parteck M200, sold by Merck®) | 71.86 | 35.92 |
| Crosslinked polyvinylpyrrolidone (Kollidon® CL, sold by ISP®) | 20.00 | 10.00 |
| Aspartame (sold by Ajinomoto sweeteners Europe SAS) | 3.00 | 1.50 |
| Sodium stearyl fumarate Pruv®, sold by JRS Pharma®) | 3.00 | 1.50 |
| Titanium dioxide (Titanium Dioxide 3328®, sold by Cognis®) | 30.00 | 15.00 |
| Microcrystalline cellulose (Avicel® PH 101, sold by FMC Biopolymer®) | 20.00 | 10.00 |
| Glyceryl behenate (Compritol 888 ATO®, sold by Gattefosse®) | 6.00 | 3.00 |
| Calcium carbonate (Hubercal® 150, sold by Huber®) | 30.00 | 15.00 |
| Total | 200.00 | 100.00 |

Breaking up was measured according to the test described in section 2.9.1 of the European Pharmacopoeia using a device of Sotax DT3 type. The test was carried out with a disc. The result obtained is 14 s.

### Example 3

### Orodispersible zolpidem tartrate tablets, dosage 2.5 mg

The orodispersible zolpidem tartrate tablets, dosed at 2.5 mg, are manufactured according to the same manufacturing process as that described in Example 1.

The composition is described in detail in the following Table 4.

**Table 4**

| **Composition** | **Unit formula (mg)** | **Percentage formula (%)** |
|---|---|---|
| Zolpidem tartrate | 2.50 | 1.67 |
| Anhydrous colloidal silica (Aerosil® 200, sold by Evonik Degussa GmbH®) | 0.05 | 0.03 |
| Amino-methacrylate copolymer(Eudragit® EPO, sold by Evonik Degussa GmbH®) | 3.47 | 2.32 |
| Sodium lauryl sulphate (sold by Cognis®) | 0.34 | 0.23 |
| Stearic acid (Stearic Acid 2236®, sold by Mallinckrodt Baker®) | 0.52 | 0.34 |
| Hydrated colloidal silica (Syloïd® FP 244, sold by GRACE Davison®) | 1.20 | 0.80 |
| Mannitol (Pearlitol® 160C, sold by Roquette®, and/or Parteck M200, sold by Merck®) | 50.40 | 33.6 |
| Crosslinked polyvinylpyrrolidone (Kollidon® CL, sold by ISP®) | 15.00 | 10.00 |
| Aspartame (sold by Ajinomoto sweeteners Europe SAS) | 2.25 | 1.50 |
| Sodium stearyl fumarate (Pruv®, sold by JRS Pharma®) | 2.25 | 1.50 |
| Titanium dioxide (Titanium Dioxide 3328®, sold by Cognis®) | 30.00 | 15,00 |
| Microcrystalline cellulose (Avicel® PH 101, sold by FMC Biopolymer®) | 15.00 | 10.00 |
| Glyceryl behenate (Compritol 888 ATO®, sold by Gattefosse®) | 4.50 | 3.00 |
| Calcium carbonate (Hubercal® 150, sold by Huber®) | 22.5 | 15.00 |
| Total | 150.00 | 100.00 |

Breaking up was measured according to the test described in section 2.9.1 of the European Pharmacopoeia using a device of Sotax DT3 type. The test was carried out without a disc. The result obtained is 14 s.

### Example 4

### Film-coated zolpidem tartrate tablets, dosage 10 mg

### Manufacturing process:

Zolpidem tartrate, lactose, microcrystalline cellulose and sodium carboxymethylstarch are mixed in a mixer-granulator and then granulated with an aqueous hypromellose-based solution. The granule obtained is subsequently transferred into a fluidized bed and dried. The dry granule is then graded.

The graded particle obtained is lubricated with magnesium stearate in a tumbler mixer and then the antimisuse agents, which are titanium dioxide, glyceryl behenate, the colorant and calcium carbonate, are added to the mixture. The combination is then homogenized in a tumbler mixer.

Finally, the mixture obtained is tabletted on a rotary tabletting press.

Breaking up was measured according to the test described in section 2.9.1 of the European Pharmacopoeia using a device of Sotax DT3 type. The test was carried out with a disc. The result obtained is 1 min 12 s.

The composition is described in detail in the following Table 5.

**Table 5**

| **Composition** | **Unit formula (mg)** | **Percentage formula (%)** |
|---|---|---|
| Zolpidem tartrate | 10.00 | 4.42 |
| Hypromellose 6 cp (Pharmacoat® 606, sold by ShinEtsu®) | 2.50 | 1.11 |
| Lactose 150 mesh EUR/US (Pharmatose® 150M, sold by DMV-Fonterra Excipients®) | 90.40 | 40.00 |
| Microcrystalline cellulose (Avicel® PH 101, sold by FMC Biopolymer® | 12.10 | 5.35 |
| Sodium carboxymethylstarch (Primojel®, sold by Avebe®) | 3.80 | 1.68 |
| Magnesium stearate (sold by Mallinckrodt Chemical®) | 1.20 | 0.53 |
| **Granule** | **120.00** | **53.09** |
| **SUBTOTAL** | | |
| Titanium dioxide (Titanium Dioxide 3328®, sold by Cognis®) | 28.00 | 12.39 |
| Glyceryl behenate (Compritol 888 ATO®, sold by Gattefosse®) | 7.00 | 3.10 |
| Brilliant blue colorant | 20.00 | 8.85 |
| Calcium carbonate (Hubercal® 150, sold by Huber®) | 35.00 | 15.49 |
| **Core** | **210.00** | **92.92** |
| **SUBTOTAL** | | |
| Opadry 2® Yellow 32F32349 (sold by Colorcon®), | | |
| including: | | |
| - Lactose monohydrate: 36.00% w/w | | |
| - HPMC 2910/hypromellose 15 cP: 28.00% w/w | 16.00 | 7.08 |
| - Titanium dioxide: 24.83% w/w | | |
| - Macrogol/PEG 3350: 10.00 % w/w | | |
| - Yellow iron oxide: 1.17% w/w | | |
| TOTAL, film-coated tablet | 226.00 | 100.00 |

The antimisuse effectiveness was evaluated by introducing a tablet into different drinks. The results obtained are presented in the following Table 6.

**Table 6**

| **Drink evaluated** | **Appearance of the drink after introduction of the tablet** | **Visual detection of the tablet** |
|---|---|---|
| Water | Cloudy liquid with blue suspended particles | Yes |
| Orange juice | Change in colour of the orange juice to green | Yes |
| Fizzy drink of cola type | Significant effervescence of the fizzy drink with presence of blue particles in the foam | Yes |
| Wine | Presence of white particles floating at the surface of the liquid | Yes |
| Whisky | Cloudiness of the liquid with greenish coloration | Yes |

### Example 5

Orodispersible zolpidem tartrate tablets, dosages 2.5 mg, 5.0 mg and 10.0 mg The orodispersible zolpidem tartrate tablets thus dosed are manufactured according to the same manufacturing process as that described in Example 1.

The compositions are described in detail in the following Tables 7, 8 and 9.

**Table 7**

| **Composition** | **Unit formula (mg)** | **Percentage formula (%)** |
|---|---|---|
| Zolpidem tartrate | 2.50 | 1.67 |
| Anhydrous colloidal silica (Aerosil® 200, sold by Evonik Degussa GmbH®) | 0.05 | 0.05 |
| Amino-methacrylate copolymer (Eudragit® EPO, sold by Evonik Degussa GmbH®) | 2.84 | 2.84 |
| Sodium lauryl sulphate (sold by Cognis®) | 0.29 | 0.29 |
| Stearic acid (Stearic Acid 2236®, sold by Mallinckrodt Baker®) | 0.42 | 0.42 |
| Hydrated colloidal silica (Syloid® FP 244, sold by Grace Davison®) | 0.98 | 0.98 |
| Zolpiden tartrate granule subtotal | 7.08 | 7.08 |
| Mannitol powder (Pearlitol® 160C, sold by Roquette®) | 25.60 | 25.60 |
| Mannitol granules (Parteck M200, sold by Merck®) | 17.07 | 17.14 |
| Crosslinked polyvinylpyrrolidone (Kollidon® CL, sold by ISP®) | 7.00 | 7.00 |
| Aspartame (sold by Ajinomoto Sweeteners Europe SAS) | 1.50 | 1.50 |
| Sodium stearyl fumarate (Pruv®, sold by JRS Pharma®) | 1.50 | 1.50 |
| Titanium Dioxide (Titanium Dioxide 3328®, sold by Cognis®) | 15.00 | 15.00 |
| Microcrystalline cellulose (Avicel® PH 101, sold by FMC Biopolymer®) | 10.00 | 10.00 |
| Glyceryl behenate (Compritol 888 ATO®, sold by Gattefosse®) | 0.25 | 0.25 |
| Calcium carbonate (Hubercal® 150, sold by Huber®) | 15.00 | 15.00 |
| Total | 100.00 | 100.00 |

**Table 8**

| **Composition** | **Unit formula (mg)** | **Percentage formula (%)** |
|---|---|---|
| Zolpidem tartrate | 5.00 | 2.50 |
| Anhydrous colloidal silica (Aerosil® 200, sold by Evonik Degussa GmbH®) | 0.10 | 0.05 |
| Amino-methacrylate copolymer (Eudragit® EPO, sold by Evonik Degussa GmbH®) | 5.69 | 2.85 |
| Sodium lauryl sulphate (sold by Cognis®) | 0.57 | 0.29 |
| Stearic acid (Stearic Acid 2236®, sold by Mallinckrodt Baker®) | 0.84 | 0.42 |
| Hydrated colloidal silica (Syloid® FP 244, sold by Grace Davison®) | 1.97 | 0.99 |
| Zolpiden tartrate granule subtotal | 14.17 | 7.09 |
| Mannitol powder (Pearlitol® 160C, sold by Roquette®) | 51.20 | 25.60 |
| Mannitol granules (Parteck M200, sold by Merck®) | 34.13 | 17.07 |
| Crosslinked polyvinylpyrrolidone (Kollidon® CL, sold by ISP®) | 14.00 | 7.00 |
| Aspartame (sold by Ajinomoto Sweeteners Europe SAS) | 3.00 | 1.50 |
| Sodium stearyl fumarate (Pruv®, sold by JRS Pharma®) | 3.00 | 1.50 |
| Titanium Dioxide (Titanium Dioxide 3328®, sold by Cognis®) | 30.00 | 15.00 |
| Microcrystalline cellulose (Avicel® PH 101, sold by FMC Biopolymer®) | 20.00 | 10.00 |
| Glyceryl behenate (Compritol 888 ATO®, sold by Gattefosse®) | 0.50 | 0.25 |
| Calcium carbonate (Hubercal® 150, sold by Huber®) | 30.00 | 15.00 |
| Total | 200.00 | 100.00 |

**Table 9**

| **Composition** | **Unit formula (mg)** | **Percentage formula (%)** |
|---|---|---|
| Zolpidem tartrate | 10.00 | 2.50 |
| Anhydrous colloidal silica (Aerosil® 200, sold by Evonik Degussa GmbH®) | 0.20 | 0.05 |
| Amino-methacrylate copolymer (Eudragit® EPO, sold by Evonik Degussa GmbH®) | 11.37 | 2.84 |
| Sodium lauryl sulphate (sold by Cognis®) | 1.14 | 0.29 |
| Stearic acid (Stearic Acid 2236®, sold by Mallinckrodt Baker®) | 1.69 | 0.42 |
| Hydrated colloidal silica (Syloid® FP 244, sold by Grace Davison®) | 3.93 | 0.98 |
| Zolpiden tartrate granule subtotal | 28.33 | 7.08 |
| Mannitol powder (Pearlitol® 160C, sold by Roquette®) | 102.40 | 25.60 |
| Mannitol granules (Parteck M200, sold by Merck®) | 68.26 | 17.07 |
| Crosslinked polyvinylpyrrolidone (Kollidon® CL, sold by ISP®) | 28.00 | 7.00 |
| Aspartame (sold by Ajinomoto Sweeteners Europe SAS) | 6.00 | 1.50 |
| Sodium stearyl fumarate (Pruv®, sold by JRS Pharma®) | 6.00 | 1.50 |
| Titanium Dioxide (Titanium Dioxide 3328®, sold by Cognis®) | 60.00 | 15.00 |
| Microcrystalline cellulose (Avicel® PH 101, sold by FMC Biopolymer®) | 40.00 | 10.00 |
| Glyceryl behenate (Compritol 888 ATO®, sold by Gattefosse®) | 1.00 | 0.25 |
| Calcium carbonate (Hubercal® 150, sold by Huber®) | 60.00 | 15.00 |
| Total | 400.00 | 100.00 |

Breaking up was measured for these tablets according to the test described in section 2.9.1 of the European Pharmacopoeia, using a device of Sotax DT3 type. The test was carried out without the disc. The results obtained are 17 s approximately for these three compositions.

The antimisuse effectiveness was evaluated by introducing these tablets into different drinks. The results obtained are similar for the three compositions. The results thus obtained for the three compositions are presented in the following Table 10.

**Table 10**

| **Drink evaluated** | **Appearance of the drink after introduction of the tablet** | **Visual detection of the tablet** |
|---|---|---|
| Water | Cloudy liquid with blue suspended particles | Yes |
| Orange juice | Change in colour of the orange juice to green | Yes |
| Fizzy drink of cola type | Significant effervescence of the fizzy drink with presence of blue particles in the foam | Yes |
| Wine | Presence of white particles floating at the surface of the liquid | Yes |
| Whisky | Cloudiness of the liquid with greenish coloration | Yes |

### Example 6

### Orodispersible zopiclone tablets, dosage 7.5 mg

The orodispersible zopiclone tablets, dosed at 7.5 mg, are manufactured according to the same manufacturing process as that described in Example 1.

The composition is described in detail in the following Table 11.

**Table 11**

| **Composition** | **Percentage formula (%)** | **Unit formula (mg)** |
|---|---|---|
| Zopiclone | 3.00 | 7.5 |
| Mannitol powder (Pearlitol® 160C, sold by Roquette®) | 28.05 | 70.125 |
| Mannitol granules (Parteck M200, sold by Merck®) | 18.70 | 46.750 |
| Crosslinked polyvinylpyrrolidone (Kollidon® CL, sold by ISP®) | 7.00 | 17.500 |
| Aspartame (sold by Ajinomoto Sweeteners Europe SAS) | 1.50 | 3.750 |
| Sodium stearyl fumarate (Pruv®, sold by JRS Pharma®) | 1.50 | 3.750 |
| Titanium dioxide (Titanium Dioxide 3328®) sold by Cognis®) | 15.00 | 37.500 |
| Microcrystalline cellulose (Avicel® PH 101, sold by FMC Biopolymer®) | 10.00 | 25.000 |
| Glyceryl behenate (Compritol 888 ATO® sold by Gattefosse®) | 0.25 | 0.625 |
| Calcium carbonate (Hubercal® 150, sold by Huber®) | 15.00 | 37.500 |
| Total | 100.00 | 250.00 |

The antimisuse effectiveness was evaluated by introducing these tablets into different drinks. The results obtained are presented in the following Table 12.

**Table 12**

| **Drink evaluated** | **Appearance of the drink after introduction of the tablet** | **Visual detection of the tablet** |
|---|---|---|
| Water | Cloudy liquid with blue suspended particles | Yes |
| Orange juice | Change in colour of the orange juice to green | Yes |
| Fizzy drink of cola type | Significant effervescence of the fizzy drink with presence of blue particles in the foam | Yes |
| Wine | Presence of white particles floating at the surface of the liquid | Yes |
| Whisky | Cloudiness of the liquid with greenish coloration | Yes |

## Claims

1. Pharmaceutical composition for oral administration, **characterized in that** it comprises an active principle which might form the object of an abuse of use or one of its pharmaceutically acceptable salts and **in that** it comprises components which, if the composition is introduced into an aqueous drink optionally comprising alcohol, generate visual means on contact with the latter, the said visual means corresponding to an opacification of the drink, to the formation of insoluble particles and to at least one other visual means chosen from effervescence and the flotation of the tablet and a combination of these visual means, wherein the components generating the said opacification of the drink comprises titanium dioxide particles and wherein components providing the said formation of insoluble particles comprises glyceryl behenate.

2. Pharmaceutical composition according to Claim 1, **characterized in that** the components generating the opacification of the drink consist of titanium dioxide particles.

3. Pharmaceutical composition according to either of Claims 1 and 2, **characterized in that** it comprises at least 15 mg, preferably at least 20 mg, of titanium dioxide particles.

4. Pharmaceutical composition according to one of Claims 1 to 3, **characterized in that** it comprises at least one effervescence generator.

5. Composition according to Claim 4, **characterized in that** the effervescence generator is composed solely of a carbon dioxide generator, preferably chosen from a carbonate or a bicarbonate of an alkali metal, of an alkaline earth metal or of an amino acid, such as calcium carbonate, sodium bicarbonate, potassium bicarbonate, potassium carbonate, L-lysine carbonate, arginine carbonate, sodium sesquicarbonate and their mixture; advantageously, the carbon dioxide generator is calcium carbonate.

6. Pharmaceutical composition according to any one of the preceding claims, **characterized in that** it additionally comprises at least one disintegrating agent preferably chosen from sodium carboxymethylstarch, crosslinked sodium carboxymethylcellulose and crosslinked polyvinylpyrrolidone, and more advantageously crosslinked polyvinylpyrrolidone.

7. Pharmaceutical composition according to any one of the preceding claims, **characterized in that** it additionally comprises at least one soluble agent of polyol type preferably chosen from mannitol, xylitol, sorbitol, maltitol and their mixture.

8. Pharmaceutical composition according to Claim 7, **characterized in that** the amount of the soluble agent of polyol type is between 20 and 50% by weight, preferably between 30 and 45% by weight, with respect to the total weight of the composition.

9. Pharmaceutical composition according to one of Claims 6 to 8, **characterized in that** the composition is for rapid disintegration in the mouth.

10. Pharmaceutical composition according to any one of Claims 1 to 9, **characterized in that** the active principle is coated with a coating agent based on a polymer which is soluble at pH 5 or less and which is permeable at pH above 5.

11. Pharmaceutical composition according to any one of the preceding claims, **characterized in that** it additionally comprises at least one hydrophilic excipient preferably chosen from cellulose, hydroxyethylcellulose, hydroxypropylcellulose, hydroxypropylmethylcellulose, carboxymethylcellulose or sodium carboxymethylcellulose derivatives; vegetable gums and their derivatives; alginic acid derivatives; polyethylene glycols and their derivatives; starches and their derivatives; silicas and their derivatives; polymethacrylates; and copolymers of acrylic and methacrylic acids.

12. Pharmaceutical composition according to one of the preceding claims, **characterized in that** it is provided in the form of a tablet.

13. Pharmaceutical composition according to any one of Claims 1 to 12, **characterized in that** the active principle which might form the object of an abuse of use is a hypnotic compound; the hypnotic compound preferably belongs to the category of the benzodiazepines, cyclopyrrolones, pyrazolopyrimidines or imidazopyridines.

14. Pharmaceutical tablet according to claim 1 comprising **(1)** an internal phase comprising coated granules based on an active principle which might form the object of an abuse of use or one of its pharmaceutically acceptable salts, in particular a hypnotic compound, and a coating agent based on a polymer which is soluble at pH 5 or less and which is permeable at a pH above 5, **(2)** an external phase comprising components which, if the tablet is introduced into an aqueous drink which optionally comprises alcohol, generate visual means corresponding to an opacification of the drink and to at least one other visual means chosen from effervescence of the tablet, the formation of insoluble particles, the flotation of the tablet and a combination of these visual means, the components generating the said opacification of the drink comprising titanium dioxide particles, preferably consisting of titanium dioxide particles, and the said external phase additionally comprising at least one disintegrating agent and optionally at least one soluble agent of polyol type.

## Patentansprüche

1. Pharmazeutische Zusammensetzung zur oralen Verabreichung, **dadurch gekennzeichnet, dass** sie ein Wirkprinzip, das den Gegenstand eines Verwendungsmissbrauchs bilden kann, oder eines seiner pharmazeutisch akzeptablen Salze umfasst, und dass sie Komponenten umfasst, die, wenn die Zusammensetzung in ein wässriges Getränk eingebracht wird, das gegebenenfalls Alkohol umfasst, visuelle Mittel bei Kontakt mit dem Letzteren erzeugen, wobei die visuellen Mittel einer Trübung des Getränks, der Bildung unlöslicher Partikel und mindestens einem anderen visuellen Mittel entsprechen, das aus Aufschäumen und der Flotation der Tablette und einer Kombination dieser visuellen Mittel ausgewählt ist, wobei die Komponenten, die die Trübung des Getränks hervorrufen, Titandioxidpartikel umfassen und wobei die Komponenten, die die Bildung unlöslicher Partikel herbeiführen, Glycerylbehenat umfassen.

2. Pharmazeutische Zusammensetzung nach Anspruch 1, **dadurch gekennzeichnet, dass** die Komponenten, die die Trübung des Getränks hervorrufen, aus Titandioxidpartikeln bestehen.

3. Pharmazeutische Zusammensetzung nach einem der Ansprüche 1 und 2, **dadurch gekennzeichnet, dass** sie mindestens 15 mg, vorzugsweise mindestens 20 mg Titandioxidpartikel umfasst.

4. Pharmazeutische Zusammensetzung nach einem der Ansprüche 1 bis 3, **dadurch gekennzeichnet, dass** sie mindestens einen Schaumbildner umfasst.

5. Zusammensetzung nach Anspruch 4, **dadurch gekennzeichnet, dass** der Schaumbildner nur aus einem Kohlendioxidbildner besteht, der vorzugsweise ausgewählt ist aus einem Carbonat oder einem Bicarbonat eines Alkalimetalls, eines Erdalkalimetalls oder einer Aminosäure, wie Calciumcarbonat, Natriumbicarbonat, Kaliumbicarbonat, Kaliumcarbonat, L-Lysincarbonat, Arginincarbonat, Natriumsesquicarbonat und ihrem Gemisch; vorteilhafterweise ist der Kohlendioxidbildner Calciumcarbonat.

6. Pharmazeutische Zusammensetzung nach einem der vorhergehenden Ansprüche, **dadurch gekennzeichnet, dass** sie zusätzlich mindestens ein Aufschlussmittel umfasst, das vorzugsweise ausgewählt ist aus Natriumcarboxymethylstärke, vernetzter Natriumcarboxymethylcellulose und vernetztem Polyvinylpyrrolidon und besonders vorteilhaft vernetztem Polyvinylpyrrolidon.

7. Pharmazeutische Zusammensetzung nach einem der vorhergehenden Ansprüche, **dadurch gekennzeichnet, dass** sie zusätzlich mindestens ein lösliches Mittel vom Polyoltyp umfasst, das vorzugsweise aus Mannit, Xylit, Sorbit, Maltit und ihrem Gemisch ausgewählt ist.

8. Pharmazeutische Zusammensetzung nach Anspruch 7, **dadurch gekennzeichnet, dass** die Menge des löslichen Mittels vom Polyoltyp zwischen 20 und 50 Gew.-%, vorzugsweise zwischen 30 und 45 Gew.-%, bezogen auf das Gesamtgewicht der Zusammensetzung, beträgt.

9. Pharmazeutische Zusammensetzung nach einem der Ansprüche 6 bis 8, **dadurch gekennzeichnet, dass** die Zusammensetzung zum schnellen Aufschließen im Mund vorgesehen ist.

10. Pharmazeutische Zusammensetzung nach einem der Ansprüche 1 bis 9, **dadurch gekennzeichnet, dass** das Wirkprinzip mit einem Überzugsmittel auf der Basis eines Polymers beschichtet ist, das bei einem pH-Wert von 5 oder darunter löslich ist und das bei einem pH-Wert über 5 permeabel ist.

11. Pharmazeutische Zusammensetzung nach einem der vorhergehenden Ansprüche, **dadurch gekennzeichnet, dass** sie zusätzlich mindestens einen hydrophilen Exzipienten umfasst, der vorzugsweise ausgewählt ist aus Cellulose, Hydroxyethylcellulose, Hydroxypropylcellulose, Hydroxypropylmethylcellulose, Carboxymethylcellulose oder Natriumcarboxymethylcellulosederivaten; pflanzlichen Gummis und ihren Derivaten; Alginsäurederivaten; Polyethylenglykolen und ihren Derivaten; Stärken und ihren Derivaten; Kieselsäuren und ihren Derivaten; Polymethacrylaten; und Copolymeren von Acryl- und Methacrylsäuren.

12. Pharmazeutische Zusammensetzung nach einem der vorhergehenden Ansprüche, **dadurch gekennzeichnet, dass** sie in Form einer Tablette bereitgestellt wird.

13. Pharmazeutische Zusammensetzung nach einem der Ansprüche 1 bis 12, **dadurch gekennzeichnet, dass** das Wirkprinzip, das den Gegenstand eines Verwendungsmissbrauchs bilden kann, eine Schlafmittelverbindung ist; die Schlafmittelverbindung gehört vorzugsweise zu der Kategorie der Benzodiazepine, Cyclopyrrolone, Pyrazolopyrimidine oder Imidazopyridine.

14. Pharmazeutische Tablette nach Anspruch 1, umfassend **(1)** eine innere Phase, umfassend beschichtete Granulate auf der Basis eines Wirkprinzips, das den Gegenstand eines Verwendungsmissbrauchs bilden kann, oder eines seiner pharmazeutisch akzeptablen Salze, insbesondere eine Schlafmittelverbindung, und ein Überzugsmittel auf der Basis eines Polymers, das bei einem pH-Wert von 5 oder weniger löslich ist und das bei einem pH-Wert über 5 permeabel ist, **(2)** eine äußere Phase, die Komponenten umfasst, die, wenn die Tablette in ein wässriges Getränk eingebracht wird, das gegebenenfalls Alkohol enthält, visuelle Mittel erzeugen, die einer Trübung des Getränks und mindestens einem weiteren visuellen Mittel entsprechen, ausgewählt aus dem Aufschäumen der Tablette, der Bildung unlöslicher Partikel, der Flotation der Tablette und einer Kombination dieser visuellen Mittel, wobei die Komponenten, die die Trübung des Getränks hervorrufen, Titandioxidpartikel umfassen, vorzugsweise aus Titandioxidpartikeln bestehen, und wobei die äußere Phase zusätzlich mindestens ein Aufschlussmittel und gegebenenfalls mindestens ein lösliches Mittel vom Polyoltyp umfasst.

## Revendications

1. Composition pharmaceutique pour administration orale, **caractérisée en ce qu'**elle comprend une substance active qui pourrait faire l'objet d'une utilisation abusive ou l'un de ses sels pharmaceutiquement acceptables et **en ce qu'**elle comprend des composants qui, si la composition est introduite dans une boisson aqueuse comprenant facultativement de l'alcool, génèrent des moyens visuels au contact de cette dernière, lesdits moyens visuels correspondant à une opacification de la boisson, à la formation de particules insolubles et à au moins un autre moyen visuel choisi parmi l'effervescence et la flottation du comprimé et une combinaison de ces moyens visuels, dans laquelle les composants générant ladite opacification de la boisson comprennent des particules de dioxyde de titane et dans laquelle les composants produisant ladite formation de particules insolubles comprennent du béhénate de glycéryle.

2. Composition pharmaceutique selon la revendication 1, **caractérisée en ce que** les composants générant l'opacification de la boisson sont constitués de particules de dioxyde de titane.

3. Composition pharmaceutique selon l'une des revendications 1 et 2, **caractérisée en ce qu'**elle comprend au moins 15 mg, de préférence au moins 20 mg, de particules de dioxyde de titane.

4. Composition pharmaceutique selon une des revendications 1 à 3, **caractérisée en ce qu'**elle comprend au moins un générateur d'effervescence.

5. Composition selon la revendication 4, **caractérisée en ce que** le générateur d'effervescence est composé exclusivement de générateur de dioxyde de carbone, de préférence choisi parmi un carbonate ou un bicarbonate d'un métal alcalin, d'un métal alcalino-terreux ou d'un acide aminé, tel que le carbonate de calcium, le bicarbonate de sodium, le bicarbonate de potassium, le carbonate de potassium, le carbonate de L-lysine, le carbonate d'arginine, le sesquicarbonate de sodium et leur mélange ; avantageusement, le générateur de dioxyde de carbone est le carbonate de calcium.

6. Composition pharmaceutique selon l'une quelconque des revendications précédentes, **caractérisée en ce qu'**elle comprend en outre au moins un agent délitant, de préférence choisi parmi le carboxyméthylamidon sodique, la carboxyméthylcellulose sodique réticulée et la polyvinylpyrrolidone réticulée, et plus avantageusement la polyvinylpyrrolidone réticulée.

7. Composition pharmaceutique selon l'une quelconque des revendications précédentes, **caractérisée en ce qu'**elle comprend en outre au moins un agent soluble de type polyol, de préférence choisi parmi le mannitol, le xylitol, le sorbitol, le maltitol et leur mélange.

8. Composition pharmaceutique selon la revendication 7, **caractérisée en ce que** la quantité de l'agent soluble de type polyol est comprise entre 20 et 50 % en poids, de préférence entre 30 et 45 % en poids, par rapport au poids total de la composition.

9. Composition pharmaceutique selon une des revendications 6 à 8, **caractérisée en ce que** la composition est pour désintégration rapide dans la bouche.

10. Composition pharmaceutique selon l'une quelconque des revendications 1 à 9, **caractérisée en ce que** la substance active est enrobée avec un agent d'enrobage à base de polymère qui est soluble à pH 5 ou moins et qui est perméable à un pH supérieur à 5.

11. Composition pharmaceutique selon l'une quelconque des revendications précédentes, **caractérisée en ce qu'**elle comprend en outre au moins un excipient hydrophile, de préférence choisi parmi la cellulose, l'hydroxyéthylcellulose, l'hydroxypropylcellulose, l'hydroxypropylméthylcellulose, la carboxyméthylcellulose ou des dérivés de carboxyméthylcellulose sodique ; des gommes végétales et leurs dérivés ; des dérivés d'acide alginique ; des polyéthylène glycols et leurs dérivés ; des amidons et leurs dérivés ; des silices et leurs dérivés ; des polyméthacrylates ; et des copolymères d'acides acrylique et méthacrylique.

12. Composition pharmaceutique selon une des revendications précédentes, **caractérisée en ce qu'**elle est fournie sous la forme d'un comprimé.

13. Composition pharmaceutique selon l'une quelconque des revendications 1 à 12, **caractérisée en ce que** la substance active qui peut faire l'objet d'une utilisation abusive est un composé hypnotique ; le composé hypnotique appartient de préférence à la catégorie des benzodiazépines, des cyclopyrrolones, des pyrazolopyrimidines ou des imidazopyridines.

14. Comprimé pharmaceutique selon la revendication 1 comprenant (1) une phase interne comprenant des granules enrobés à base d'une substance active qui peut faire l'objet d'une utilisation abusive ou l'un de ses sels pharmaceutiquement acceptables, en particulier un composé hypnotique, et un agent d'enrobage à base de polymère qui est soluble à pH 5 ou moins et qui est perméable à un pH supérieur à 5, (2) une phase externe comprenant des composants qui, si le comprimé est introduit dans une boisson aqueuse qui comprend facultativement de l'alcool, génèrent des moyens visuels correspondant à une opacification de la boisson et à au moins un autre moyen visuel choisi parmi l'effervescence du comprimé, la formation de particules insolubles, la flottation du comprimé et une combinaison de ces moyens visuels, les composants générant ladite opacification de la boisson comprenant des particules de dioxyde de titane, de préférence étant constitués de particules de dioxyde de titane, et ladite phase externe comprenant en outre au moins un agent délitant et facultativement, au moins un agent soluble de type polyol.
